# EUROPÄISCHE PATENTSCHRIFT

(11) **EP 0 998 222 B1**
(45) Veröffentlichungstag und Bekanntmachung des Hinweises auf die Patenterteilung: **28.04.2004**
(21) Anmeldenummer: 98945127.3
(22) Anmeldetag: 31.07.1998
(51) Int. Cl.: A61B 8/06

(54) **VORRICHTUNG UND VERFAHREN ZUR EMBOLIEDETEKTION**
DEVICE AND METHOD FOR DETECTING EMBOLI
DISPOSITIF ET PROCEDE POUR LA DETECTION D'EMBOLIES

(30) Priorität: 31.07.1997 DE 19733091
(43) Veröffentlichungstag der Anmeldung: 10.05.2000
(73) Patentinhaber: DWL Elektronische Systeme GmbH, 78224 Singen (DE)
(72) Erfinder: BRUCHER, Rainer, D-89173 Lonsee (DE); DENNER, Dieter, D-78354 Sipplingen (DE)
(74) Vertreter: Behrmann, Niels, Dipl.-Ing.
(86) Internationale Anmeldenummer: PCT/EP1998/004786
(87) Internationale Veröffentlichungsnummer: WO 1999/005970

(56) Entgegenhaltungen:
- WO-A-92/07508
- WO-A-94/06353
- US-A- 3 762 221
- US-A- 5 738 097

## Beschreibung

Die vorliegende Erfindung betrifft eine Vorrichtung zur Emboliedetektion nach dem Oberbegriff des Patentanspruchs 1 (wie aus WO-A-9 406 353 bekannt) sowie ein Verfahren zur Emboliedetektion.

Derartige gattungsgemäße Vorrichtungen bzw. Verfahren werden in der medizinischen Ultraschall-Diagnostik eingesetzt, um mit Hilfe der reflektierten Ultraschall-Dopplersignale und insbesondere Änderungen von diesen Rückschlüsse auf Fremdkörper ziehen zu können, die sich in einem Blutgefäß befinden. Bekannt ist, daß insbesondere Embolien oder vergleichbare Fremdkörper sich durch ein gegenüber einem umgebenen Fluidmedium -- z. B. Blut -- stark unterschiedliches Reflektionsverhalten des eingetragenen Ultraschall-Signals auszeichnen, so daß diese Besonderheiten zur Embolieerkennung ausgenutzt werden.

Zum Stand der Technik gehören dabei Vorrichtungen, die durch eine geeignete Demodulation des empfangenen, reflektierten Dopplersignals und eine nachfolgende, akustische Ausgabe einem Bediener -- etwa einem behandelnden Arzt -eine akustische Möglichkeit geben, einen Embolus zu erkennen. Dieser äußert sich in dem akustischen Ausgabesignal durch ein charakteristisches Geräusch.

Darüber hinaus sind aus dem Stand der Technik Geräte bekannt, die ein aus einem Blutstrom reflektiertes Ultraschallsignal auch zur optischen Darstellung und Auswertung aufbereiten. Diese Vorrichtungen zeichnen sich dadurch aus, daß ein empfangenes, am Blutfluß in einem Gefäß reflektiertes Ultraschallsignal demoduliert und nachfolgend mittels einer digitalen Bildverarbeitung so aufbereitet wird, daß etwa auf einem Überwachungsmonitor eine spektrale Darstellung des Ultraschallsignals (des Bewegungssignals) über der Zeit erhältlich ist. Speziell vorgesehene Prozessoren führen diesbezüglich die notwendigen Schritte zur Bilderzeugung, insbesondere eine Fourier-Transformation der empfangenen Daten, durch. Auf diese Weise würde sich dann ein erfaßter Fremdkörper visuell auf dem Bildschirm darstellen lassen, etwa durch eine farbliche Heraushebung der charakteristischen Signalamplitude des Embolus-Signals im umgebenden Blutstrom in der spektralen Darstellung.

Im praktischen Betrieb hat es sich jedoch als nützlich herausgestellt, die Erkennung von Fremdkörpern im Blutstrom präziser vorzunehmen und insbesondere auch Embolien von sog. Artefakten, nämlich Signalstörungen einer für die gattungsgemäßen Vorrichtungen und Verfahren benutzten Ultraschallsonde -- wie sie etwa durch Bewegungen derselben entstehen -- unterscheidbar zu machen: Genau wie eine Embolie führt nämlich ein Artefakt zu einer charakteristischen Signalveränderung eines optischen oder akustischen Ausgabesignals der Vorrichtung und würde, im Falle eines Artefakten, die Diagnosegenauigkeit verschlechtern und für unnötige Ablenkung des Bedieners sorgen.

Die spezielle Aufgabe der Unterscheidung eines Embolus od. dgl. Fremdkörpers im Blutstrom von einem Artefakt wurde im Stand der Technik auf verschiedene Weisen angegangen. So macht sich etwa die Lehre der US 5,103,827 die Eigenschaften eines Artefakten (gegenüber einem Embolus) zugute, daß bei einem Artefakt in der Bilddarstellung ein bidirektionales Spektralsignal (sowohl in positiver als auch in negativer Richtung) entsteht, welches durch geeignete schaltungsund signalverarbeitungstechnische Maßnahmen von einem unidirektionalen Embolussignal unterschieden werden kann. Allerdings ist eine solche Lösung, bedingt durch die notwendigen, variablen Schwellen zur Unterscheidung und den dadurch bewirkten Aufwand, nur bedingt geeignet, einen einfachen und komfortablen Weg zur Embolus-Unterscheidung anzubieten. Auch tritt gerade bei größeren Embolien das Problem auf, daß diese -- beispielsweise durch Übersteuerung zwischengeschalteter Verstärkereinheiten -- auch zu einer bidirektionalen Spektraldarstellung führen, so daß insoweit der beschrittene Weg ohnehin zu keinem befriedigenden Ergebnis führen kann.

Ferner ist in der US 5,348,015 ein weiterer Ansatz beschrieben, einen Artefakt von einem Embolus zu unterscheiden. Insbesondere schlagen die Erfinder hier vor, eine mehrkanalige Vorrichtung, die auf verschiedenen Frequenzen betrieben wird, zur Embolusdetektion und -unterscheidung zu verwenden: Da nämlich die Ultraschall-Reflektionseigenschaften insbesondere eines Embolus frequenzabhängig sind, ist hierdurch ein sicherer Weg geschaffen, diesen etwa von einem (davon unbeeinflußten) Artefakten zu unterscheiden. In der spektralen Darstellung ist im Falle eines Embolus nämlich -- im Gegensatz zum Artefakten -- ein für verschiedene Ultraschallfrequenzen unterschiedliche Signal-(Amplituden-) Stärke festzustellen, die dann ausgewertet werden kann. Allerdings ist die in der US 5,348,015 beschriebene Vorrichtung äußerst aufwendig und macht neben mehreren Sende- und Empfangskanälen (d. h. jeweils getrennt erforderlich sind Signalerzeugung, Empfang und Demodulation) auch spezielle, für einen Mehrfrequenzbetrieb geeignete Sonden erforderlich und bietet darüber hinaus beträchtliche steuerungs- und softwaretechnische Schwierigkeiten. Insbesondere unter dem Gesichtspunkt einer kostengünstigen und wenig aufwendigen Implementierung einer zuverlässigen Emboliedetektion und -unterscheidung erscheint daher auch dieser Ansatz nachteilig.

Aufgabe der vorliegenden Erfindung ist es daher, eine gattungsgemäße Vorrichtung zur Emboliedetektion dahingehend zu verbessern, daß das Erfassen eines Embolus und insbesondere dessen Unterscheidung von einem Artefakten zuverlässiger und einfacher gestaltet werden kann. Auch ist ein entsprechendes Verfahren zur Emboliedetektion zu schaffen.

Die Aufgabe wird durch die Vorrichtung mit den Merkmalen des Patentanspruchs 1 sowie das Verfahren nach dem Patentanspruch 10 gelöst.

Vorteilhaft ermöglicht die erfindungsgemäße Vorrichtung die zuverlässige Unterscheidung eines Embolus von einem Artefakten auf der Basis zweier Signale, die hinsichtlich ihrer jeweiligen Signaländerungen -- die auf einen Embolus hinweisen könnten -- sowie des zeitlichen Abstandes zwischen diesen Signaländerungen ausgewertet werden.

Die Erfindung macht sich dabei das Lösungsprinzip zugute, daß ein Artefakt, etwa erzeugt durch Bewegung der Ultraschallsonde in der Angriffsposition am Kopf und als Störsignal in beiden auszuwertenden Signalen, i. w. gleichzeitig oder aber nur mit einem minimalen, zeitlichen Abstand auftritt. Demgegenüber wird ein Embolus dadurch unterscheidbar, daß er bei Erreichen der ersten Position im (Blut-) Gefäß die charakteristische Signaländerung -- z. B. einen Amplitudenanstieg -- hervorruft, während dies an der von der ersten Position verschiedenen, zweiten Position zu diesem Zeitpunkt noch nicht der Fall sein kann. Vielmehr entsteht an der zweiten Position, falls diese ebenfalls in demselben Gefäß vorgesehen ist, die charakteristische Signaländerung zeitlich vor oder nach der ersten Signaländerung, abhängig davon, wie die erste und die zweite Position zueinander in Flußrichtung des Fluids angeordnet sind (der Embolus bewegt sich mit der Fließgeschwindigkeit des Fluids im Gefäß), so daß der zeitliche Abstand zwischen den jeweiligen Signaländerungen der Dauer des Transports zwischen den beiden Positionen entspricht. Falls die zweite Position außerhalb des Gefäßes angeordnet ist, erfolgt im Normalfall durch den Embolus im Gefäß keine charakteristische Signaländerung des zweiten Signals.

Im Zusammenhang mit der Erfindung kann dabei die Ultraschallsendevorrichtung eine oder eine Mehrzahl von Ultraschallsonden aufweisen.

Vorteilhafte Weiterbildungen in der Erfindung sind in den Unteransprüchen beschrieben.

So ist es besonders bevorzugt, die zweite Position außerhalb des zu überwachenden Gefäßes anzuordnen; weiter bevorzugt liegt diese Position -- bei Überwachung am Kopf eines Patienten -- in einer Tiefe zwischen etwa 30 und etwa 35 mm bezogen auf die Kopfoberfläche und gerichtet auf einen Schädelknochen.

Auf diese Weise findet dann praktisch keine Signaländerung des zweiten Signals -- also im Referenzkanal -- statt, wenn ein Embolus die erste Position passiert und daraufhin das erste, charakteristische Signal erzeugt wird.

Auch ist davon auszugehen, daß der Mindest-Zeitabstand im medizinisch-praktischen Gebrauch praktisch bei Null liegt, und selbst theoretisch 2 bis 3 msec nicht übersteigt.

Weitervorteilhaft ist ein sog. Gating-System zur erfindungsgemäßen Embolieerfassung vorgesehen, nämlich ein zeitlich versetztes Auswerten desselben, reflektierten Sendesignals, wodurch das Beobachten zweier verschiedener Eindringtiefen im Körper ermöglicht wird. Im Rahmen der Erfindung wird dann eine erste Eindringtiefe auf die erste Position gelegt, während bevorzugt die die zweite Position bestimmende Eindringtiefe außerhalb des Gefäßes liegt. Auch liegt es im Rahmen der Erfindung, eine Mehrzahl von -z. B. in der Eindringtiefe abgestuften -- Gates vorzusehen, von denen dann mindestens eines als zur Erzeugung des zweiten Signals zu benutzendes Referenz-Gate einzusetzen ist.

Während üblicherweise zudem ein Betrieb der Detektoreinheit auf der Basis von in den Zeitbereich (d. h. FFT) transformierten ersten und/oder zweiten Signalen erfolgt, ist grundsätzlich im Rahmen der Erfindung auch eine Emboliedetektion und -unterscheidung im Zeitbereich möglich; vorteilhaft kann auf diese Weise zumindest für den Referenzkanal eine FFT-Prozessor eingespart werden.

Weitere Vorteile, Merkmale und Einzelheiten der Erfindung ergeben sich aus der nachfolgenden Beschreibung von Ausführungsbeispielen sowie anhand der Zeichnungen; diese zeigen in
- Fig. 1:: ein Blockschaltbild der erfindungsgemäßen Vorrichtung zur Emboliedetektion gemäß einer ersten Ausführungsform mit zwei voneinander unabhängigen Sondeneinheiten;
- Fig. 2:: eine alternative Ausführungsform der Erfindung mit einer einzelnen Sondeneinheit, für die ein Referenz-Gate vorgesehen ist;
- Fig. 3:: ein Blockschaltbild einer weiteren Ausführungsform der Erfindung, die ein Multi-Gate-System mit bis zu vier Sonden und bis zu acht beliebig den Sonden zuzuordnenden Gates vorsieht, wobei mindestens eines dieser Gates als Referenz-Gate zur Embolieunterscheidung dient, und
- Fig. 4:: ein Signaldiagramm mit einer Darstellung von drei Signalverläufen über der Zeit zur Embolieunterscheidung.

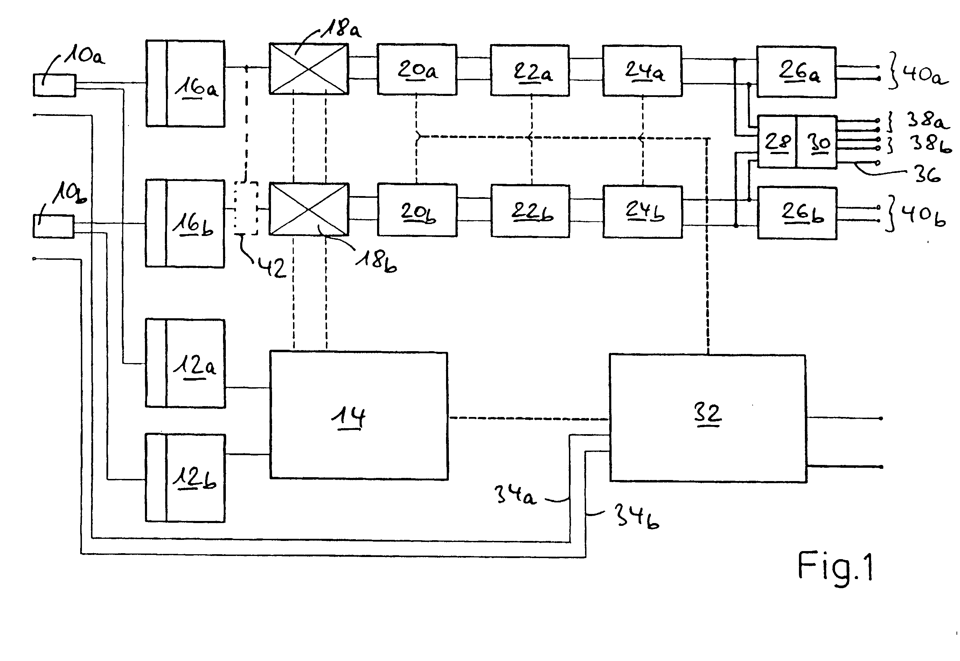

Das Blockschaltbild in Fig. 1 zeigt den Erfassungsteil einer Vorrichtung zur Emboliedetektion gemäß einer ersten, bevorzugten Ausführungsform der Erfindung (best mode). Es sind zwei voneinander unabhängige Sondenkanäle (in der Fig. mit den Indizes "a" für den ersten Kanal und "b" für den zweiten Kanal bezeichnet) vorgesehen, die jeweils getrennt für sich ein auswertbares Ultraschall-Dopplerempfangssignal erzeugen, und welches dann in der erfindungsgemäßen Weise zur Embolieunterscheidung bzw. -erkennung verwendet wird.

Eine erste Sonde 10a ist sendeseitig mit einer ersten Multiplex- und Sendeeinheit 12a verbunden, die für gängige Frequenzen der Ultraschall-Sonographie, etwa 2,4,8 oder 16 MHz eingerichtet und mit einem vorgeschalteten Oszillator-modul verbunden ist.

Empfangsseitig liegt ein Ausgangssignal der ersten Sonde 10a an einer ersten Multiplexer- und Vorverstärkereinheit 16a, wird nachfolgend von einer ersten Mischer- und Demodulatoreinheit 18a aufbereitet und einem ersten (programmierbaren) Hochpaßfilter 20a zugeleitet. Auf dieses folgt in der Anordnung des Blockschaltbildes ein erster einstellbarer Verstärker 22a ("programmable gain"), dessen Ausgangssignal von einer ersten Abtast- und Halteschaltung (Sample & Hold = S & H) 24a verarbeitet wird.

Das so entstandene Signal liegt dann zum einen einem ersten Audiofilter 26a zur Signalausgabe für ein akustisch auszuwertendes Emboliesignal an, und zum anderen empfängt eine gemeinsame FFT-Verarbeitungseinheit 28 (FFT = fast fourier transformation) das Ausgangssignal der ersten S & H-Schaltung 24a zur Vorbereitung der visuellen Darstellung des Dopplersignals als Spektraldarstellung über der Zeit. Der FFT-Verarbeitungseinheit 28 ist eine gemeinsame Detektoreinheit 30 nachgeschaltet.

Entsprechend dem beschriebenen ersten Kanal ist der mit der zweiten Sonde 10b verbundene, parallele zweite Kanal aufgebaut, und zwar mittels einer zweiten Sendeeinheit 12b, die ebenfalls durch den Oszillator 14 gesteuert wird, und empfangsseitig mittels Multiplexer/Vorverstärker 16b, Mischer 18b, Hochpaßfilter 20b, Verstärker 22b und Sample-/Hold-Schaltung 24b, deren Signal wiederum einerseits einem zweiten Audiofilter 26b zugeleitet wird, und andererseits auch an der gemeinsamen FFT-Verarbeitungseinheit 28 anliegt.

Eine gemeinsame, mittels eines Mikrocontrollers realisierte Steuereinheit 32 sorgt zum einen für eine Steuerung des ersten und zweiten Hochpasses 20a,20b, des ersten und zweiten Zwischenverstärkers 22a,22b sowie der beiden S & H-Schaltungen 24a,24b, und übernimmt darüber hinaus die Steuerung der Oszillatoreinheit 14. Diese wiederum steuert die zur Quadratur-Demodulation vorgesehenen Mischereinheiten 18a,18b. Auch ist jeder Sonde 10a,10b eine Identifikations-Signalleitung 34a,34b für die Steuereinheit 32 zugeordnet.

Mit Ausnahme der gemeinsamen FFT-Verarbeitungseinheit 28 und Detektoreinheit 30 handelt es sich bei der bislang beschriebenen Schaltung um ein gängiges Produkt, wie es von der Anmelderin etwa unter der Kennzeichnung "Multi-Dopp" als zweikanaliges System zur medizinischen Ultraschall-Diagnostik angeboten wird.

In erfindungsgemäß vorteilhafter Weise wird jedoch im Rahmen der Erfindung durch Auswertung eines jeweiligen Empfangssignals -- in dem dargestellten Ausführungsbeispiel nach einer Fourier-Transformation, also bezogen auf das Signal im Frequenzbereich -- die- Unterscheidung zwischen einem Embolus und einem zu unterdrückenden Störsignal, etwa einem Artefakt, in der Detektoreinheit 30 vorgenommen.

In der erfindungsgemäß vorteilhaften Weise ist die Detektoreinheit 30 als elektronische Einheit zur digitalen Signalverarbeitung so realisiert, daß ein Artefakt, der sich in beiden Kanälen als i. w. gleichzeitiger, deutlicher Amplitudenanstieg des Empfangssignals äußert, als ein solcher aufgrund des zeitlichen Verhältnisses zwischen den Kanälen erkannt wird und demzufolge auf einem Detektor-Signalausgang 36 kein Embolie-Erkennungssignal ausgegeben wird.

Genauer gesagt führt nämlich z. B. eine Bewegung der Sonden, die mittels einer geeigneten Trageeinheit am menschlichen Körper, etwa am Kopf, befestigt sein können, zu einem bewegungsbedingten, unerwünschten und hochpegeligen Empfangssignal in beiden Sonden, welches praktisch gleichzeitig (bzw. innerhalb eines Zeitabstandes kleiner als 3 ms) auftritt. Das entsprechend den Verarbeitungseinheiten im Blockschaltbild der Fig. 1 weiterverarbeitete Empfangssignal in beiden Kanälen ist dann durch die gemeinsame Detektoreinheit als gleichzeitig zu identifizieren, woraufhin dann der Rückschluß auf einen Artefakten erfolgt. Demgegenüber würde etwa das Auftreten eines Embolus in einem Erfassungsbereich der ersten Sonde, (die geeignet etwa auf einen Bereich eines Blutgefäßes gerichtet ist) zu einem (hochpegeligen) Signal führen, während die zweite Sonde, die auf einen anderen Bereich des Blutgefäßes, auf einen Gewebe- oder Knochenbereich außerhalb oder aber auf ein anderes Gefäß gerichtet ist (oder sich etwa im Falle einer Messung am Kopf auf dem gegenüberliegenden Ende befindet) den im ersten Kanal detektierten Embolus nicht erfassen würde. Aus diesem Grund entsteht zwar im ersten Kanal ein Erfassungssignal hoher Amplitude, nicht jedoch im zweiten Kanal (oder, im Falle eines räumlichen Versatzes der beiden Erfassungsbereiche der Sonden im selben Gefäß, im zeitlichen Abstand entsprechend der- Transportgeschwindigkeit im Blutfluß). In entsprechender Weise ist durch die Detektoreinheit die Feststellung möglich, daß in beiden Kanälen kein charakteristischer Signalanstieg zum selben Zeitpunkt erfolgt ist, so daß der Rückschluß auf einen Embolus möglich ist.

Wie in der Fig. 1 gezeigt, sind FFT-Ausgänge 38a,38b zur nachfolgenden, visuellen Signaldarstellung der transformierten Signale der Signalkanäle vorgesehen, die in der bekannten und üblichen Weise ausgebildet sind, und ergänzend kann eine behandelnde Bedienperson über Audioausgänge 40a,40b des ersten bzw. zweiten Audiofilters 26a,26b den jeweiligen Kanal akustisch überwachen.

Durch eine gestrichelt dargestellte, der zweiten Vorverstärkereinheit 16b nachgeschaltete zusätzliche Gate- und Multiplexereinheit 42 wird eine Weiterbildung der in Fig. 1 gezeigten Ausführungsform realisiert: Die zusätzliche Einheit 42 empfängt nämlich, wie durch die weitere, gestrichelte Leitung angedeutet, auch das Ausgangssignal des ersten Vorverstärkers 16a, welches die Funktion der Gate- und Multiplexereinheit 42 triggert. Die Einheit 42 bewirkt nach dem Ausgangssignal des ersten Vorverstärkes 16a eine gewisse zeitliche Verzögerung der Abtastung für den zweiten Kanal (b), wodurch -- bedingt durch die unterschiedliche Laufzeit -- ein anderer Erfassungsbereich (genauer: eine andere Erfassungstiefe) für die Signalaufbereitung im zweiten Kanal bereitsteht. Dieses so gebildete Referenzsignal (entsprechend des Zeitversatzes als "Gate" betrachtet) wird dann in der beschriebenen Weise entlang des zweiten Kanals gemischt und demoduliert (18b), gefiltert (20b), verstärkt (22b) und gesampled (24b), so daß an der gemeinsamen FFT-Verarbeitungseinheit das Ausgangssignal der Sonde 1 in zweifacher Weise -- einerseits aufbereitet durch den ersten Kanal und andererseits um einen Gate-Abstand verschoben sowie aufbereitet durch den zweiten Kanal -- anliegt.

Das Ausgangssignal des zweiten-Kanals entspricht somit einem örtlichen Versatz der Erfassungstiefe gegenüber dem ersten Kanal, wobei dieser Versatz -- je nach Einstellung der Multiplexer-/Gateeinheit 42 -- dem ersten Kanal bezogen auf eine Eindringtiefe vor- oder nachgelagert sein kann. Insbesondere kann das so realisierte Gate auch dergestalt eingestellt sein, daß das mittels Multiplexer-/Gateeinheit 42 erzeugte (Referenz-) Gatesignal nicht in das im ersten Kanal überwachte Gefäß fällt, sondern etwa auf einen Knochen oder ein dem Gefäß benachbartes Gewebe. In diesem Fall wird im zweiten, zusätzlich als Referenz verwendeten Kanal kein hochpegeliges Detektionssignal ausgegeben, wenn ein Embolus im Blutgefäß im ersten Kanal eine Signaländerung herbeiführt, so daß dann entsprechend das Vorliegen eines Embolus sicher festgestellt werden kann.

Die Fig. 4 zeigt eine übliche Laufzeit- bzw. Tiefeneinstellung, die sich insbesondere auch für den Betrieb der Vorrichtung gemäß Fig. 1 eignet. Während sowohl der Erfassungsbereich für den ersten Kanal a (oberstes Signal in der Fig. 4) als auch für den zweiten Kanal b (zweites Signal in der Fig. 4) in eine Tiefe zwischen etwa 40 und 45 mm ab Sondenoberfläche gelegt werden -- im gezeigten Ausführungsbeispiel erfolgt die Messung über die Kanäle a und b beidseits der Kopfes -- wird das Referenzsignal im ersten Kanal a_{REF} auf einen Tiefenbereich zwischen 30 und 35 mm gelegt, bei Messung am Kopf nahe den Schädelknochen des Patienten und abhängig von der jeweiligen Anatomie. Wie die Trigger- bzw. Taktsignale in Fig. 4 erkennen lassen, würde also im Falle eine Embolus -- der im Referenz-Gate nicht erfaßt werden würde, da dieses mit Positionierung auf den Knochen außerhalb des Gefäßes liegt -- nur der erste Kanal ein Embolus-Detektionssignal erzeugen, der Referenzkanal sowie der zweite jedoch nicht. Demgegenüber würde ein Artefakt gleichermaßen im Signalkanal a und im Referenzkanal a_{REF} auftreten. Das Gate erzeugt also eine Verzögerung entsprechend einer gegenüber dem Signalkanal unterschiedlichen Eindringtiefe durch die verschiedene Laufzeit.

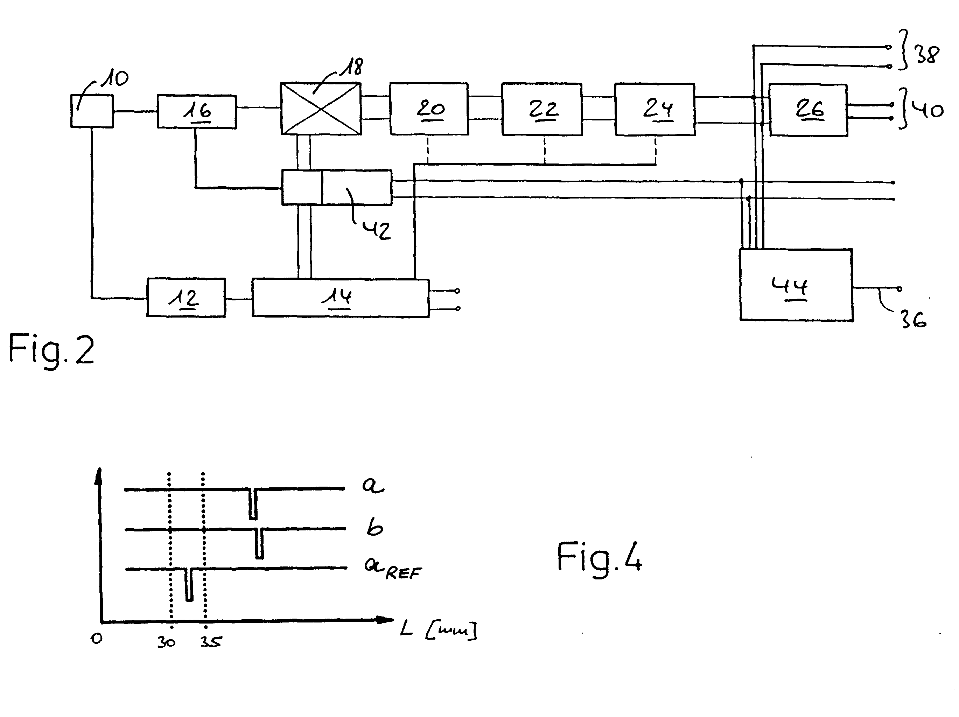

Fig. 2 zeigt im Blockschaltbild eine gegenüber der Ausführungsform in Fig. 1 leicht abgewandelte, vereinfachte Ausführungsform. Für korrespondierende Funktionselemente sind in der Fig. 2 entsprechende Bezugszeichen gewählt worden, wobei die Fig. 2 einen ersten, vollständigen Signalkanal aufweist, der endseitig wiederum einen FFT-Ausgang 38 sowie einen Audioausgang 40 besitzt, während das Referenz-Gate nicht als vollständiger Signalkanal realisiert ist, sondern lediglich als dem Vorverstärker 16 nachgeschaltete Mischer/Gateeinheit entsprechend der Einheit 42 in Fig. 1. Dieser vereinfachte Dopplerkanal benötigt nicht die weitere, nachgeschaltete Signalaufbereitung, denn er wird lediglich zur Erfassung und Unterscheidung eines Artefakten benötigt (und nicht etwa zur weiteren Berechnung und Darstellung einer fourier-transformtierten Spektralabbildung).

Dementsprechend erfaßt das Detektormodul 44 sowohl das Empfangssignal des ersten Kanals als auch das von der Gateeinheit 42 ausgegebene Signal des Referenzkanals und erzeugt daraus für den Detektor-Signalausgang 36 ein Detektorausgangssignal, wenn in der oben beschriebenen Weise ein charakteristisches Amplitudensignal am Ausgang der S & H-Schaltung 24 liegt, nicht jedoch zum selben Zeitpunkt ein entsprechendes Signal am Ausgang des Referenzgates 42.

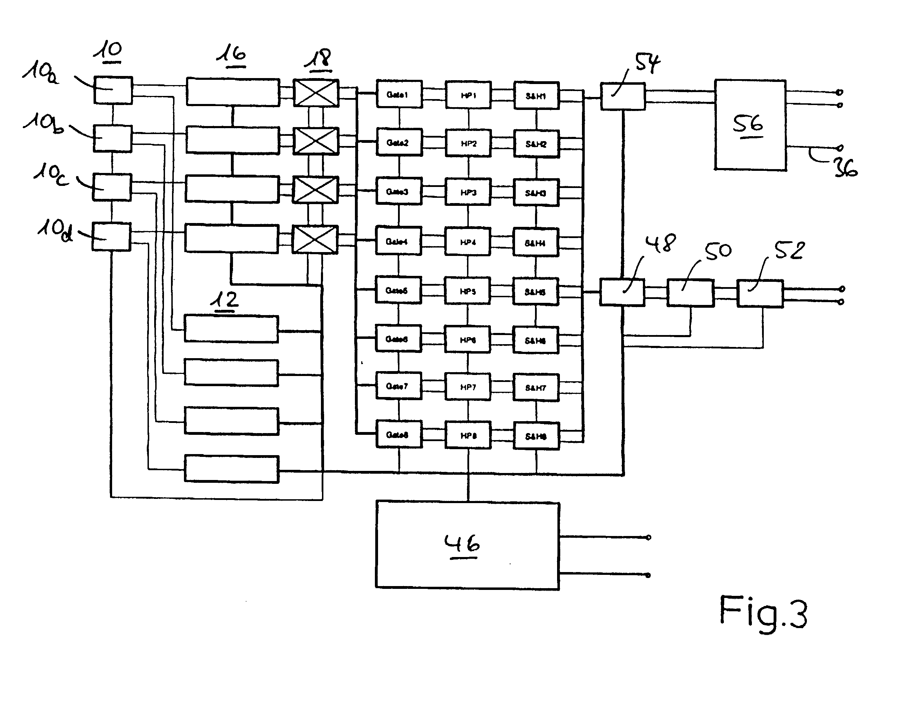

Die Fig. 3 zeigt eine weitere Ausführungsform der vorliegenden Erfindung, und zwar sind bei der Vorrichtung gemäß Fig. 3 insgesamt bis zu vier verschiedene Sonden 10a bis 10d (entsprechend einem jeweiligen von vier Kanälen a,b,c,d) anschließbar, die senderseitig von Sendeeinheiten 12a bis 12d beaufschlagt und empfangsseitig von Vorverstärkern 16a bis 16d mit nachgeschalteten Mischer/Demodulatoreinheiten 18a bis 18d abgegriffen werden. Den Mischereinheiten sind als Multiplexanordnung acht Gatekanäle, jeweils bestehend aus einem steuerbaren Gate₁, einem Hochpaß HP₁ sowie einer Abtast- und Halteschaltung S&Hᵢ (jeweils i = 1...8), nachgeschaltet. Die Steuerung und variable, individuell einstellbare Zuordnung der jeweiligen Gatekanäle zu den vier Sondenkanälen erfolgt durch Wirkung einer Gate-Steuereinheit 46, die als geeignet eingerichteter Controller realisiert ist. Durch diese Steuereinheit, die einen Oszillatorteil aufweist, werden auch die Sende- bzw. die den Gateeinheiten vorgeschalteten Empfangseinheiten gesteuert; auch der Multiplex-Audioausgang, bestehend aus Audio-Multiplexer 48, Equalizer 50 und Audioprozessor 52, sowie eine FFT-Verarbeitungseinheit 54 zur weiteren Aufbereitung und Ausgabe der Signale zur visuellen Darstellung werden von der Steuereinheit 46 mit Steuersignalen beaufschlagt.

Der FFT-Verarbeitungseinheit 54 ist wiederum eine Embolie-Detektoreinheit 56 mit einem Detektor-Signalausgang 36 nachgeschaltet, welche in der oben beschriebenen Weise aus den als Multiplex-Signal vorliegender, FFT-transformierten Gatesignalen mindestens eines der acht Gates als Referenz-Gate auswertet und aus den Signalverläufen auf das Auftreten eines Embolus (in Abgrenzung von einem Artefakt) schließt.

Die in der Fig. 3 dargestellte Ausführungsform erlaubt in sehr flexibler Weise die Verwendung von bis zu vier verschiedenen Sonden, wobei, analog der Vorgehensweise gemäß Fig. 1 oder Fig. 2, auch bereits eine einzelne Sonde den vollständigen Embolie-Erfassungsbetrieb gemäß der vorliegenden Erfindung gestattet, solange mindestens einer der acht Gatekanäle in der oben beschriebenen Weise als Referenzkanal eingerichtet ist und insbesondere auf einen abweichenden (Referenz-) Erfassungsbereich außerhalb eines überwachenden Gefäßes gerichtet ist. Die verbleibenden Gates können dann beispielsweise in gestaffelter Weise verschiedene Tiefen eines Gefäßes überwachen. Alternativ wäre eine Konfiguration denkbar, bei welcher etwa zwei Sonden mit jeweils vier tiefenweise abgestuften Gates vorgesehen sind, wobei jeweils mindestens eines der vier Gates als Referenzgate eingestellt ist.

Die in der Fig. 3 schematisch dargestellte Detektoreinheit 56 ist dann zur Signalauswertung entsprechend geeignet eingestellt bzw. programmiert.

Aus der obigen Darstellung ergibt sich, daß die Realisierung der Erfindung auf verschiedenen Wegen möglich ist, wobei es insbesondere auch nicht notwendig ist, daß vor einer Erfassung eines Embolus (verbunden mit der erfindungsgemäßen Unterscheidung von einem Artefakt) stets eine FFT-Transformation od. dgl. Operation mit dem empfangenen Ultraschallsignal erfolgen muß. Vielmehr ist es durchaus möglich, vgl. etwa die ursprüngliche Ausführungsform der Fig. 1, diese Unterscheidung allein im Zeitbereich der erfaßten bzw. zu vergleichenden Signale durchzuführen. Entsprechendes gilt für das Vorsehen von Referenz-Gates, die durch die erfindungsgemäße Änderung des Erfassungszeitpunktes des reflektierten Signals den Zustand eines (laufzeitbedingt) anderen Erfassungsbereichs im Körper widerspiegeln. Es wird davon ausgegangen, daß eine im Sinne der Erfindung beste Unterscheidung zwischen Embolie und Artefakt dann möglich ist, wenn der Erfassungsbereich für den zusätzlichen Referenzkanal an einen Ort außerhalb des zu beobachtenden Blutgefäßes gelegt wird, weil auf diese Weise die gegenseitige Beeinflussung der Kanäle weitgehend unterbleibt. In der oben skizzierten Weise ist jedoch grundsätzlich auch eine Embolieerfassung und -unterscheidung möglich, wenn die auszuwertenden Signale zwei verschiedene Erfassungsorte im selben Gefäß repräsentieren.

Insgesamt können auf die beschriebene Weise somit zuverlässig Embolien von Artefakten unterschieden werden, wobei Laborversuche gezeigt haben, daß -- verglichen mit bekannten Verfahren zur Embolieerfassung -- deutlich verbesserte Erfolgsquoten erzielt werden können.

Wird der Erfassungsbereich für den Referenzkanal auf einen Ort außerhalb des zu beobachtenden Gefäßes gelegt, so findet im Falle eines tatsächlichen Embolus im Gefäß praktisch kein Signaleintrag in den zweiten (Referenz-) Kanal statt; eventuelle Kopplungs- oder Übersprecheffekte äußern sich bestenfalls in nur geringfügig ansteigenden Amplituden im Referenzkanal.

Auch hat die meßtechnische Überprüfung ergeben, daß ein Artefakt stets praktisch gleichzeitig in beiden Kanälen auftritt und dort zu meßbaren Signalveränderungen führt; längstens beträgt eine Zeitverzögerung zwischen den Kanälen bis zu drei ms, liegt also deutlich unter den im Rahmen der vorliegenden Erfindung exemplarisch verwendeten Zeiteinstellungen

## Patentansprüche

1. Vorrichtung zur Emboliedetektion mittels an einem Fluidstrom in einem Körper reflektierten UltraschallSignalen, mit
einer zum periodischen Erzeugen von Ultraschall-Überwachungssignalen für eine geeignet eingerichtete Ultraschallsendevorrichtung und zum Empfangen eines an einer ersten Position entsprechend dem Fluidstrom in einem Gefäß reflektierten, ersten Signals ausgebildeten Ultraschalleinheit,
einer dieser nachgeschalteten Signalauswerteeinheit zum Aufbereiten und visuellen und/oder akustischen Ausgeben des reflektierten ersten Signals
und einer mit der Signalauswerteeinheit zusammenwirkenden Detektoreinheit, die zum Erfassen eines Embolus im Fluidstrom und zum Ausgeben eines Erfassungssignals als Reaktion darauf ausgebildet ist,
wobei die Ultraschalleinheit zum zusätzlichen Empfangen eines an einer zweiten Position im Körper reflektierten zweiten Signals der Ultraschall-Sendevorrichtung eingerichtet ist,
**dadurch gekennzeichnet, daß**
die Detektoreinheit zum Erfassen eines Embolus als Reaktion auf das erste und das zweite Signal dergestalt eingerichtet ist, daß ein Ausgeben des Erfassungssignals nur erfolgt, wenn eine einem möglichen Embolus entsprechende, charakteristische Signaländerung eines des ersten und zweiten Signals außerhalb eines Mindest-Zeitabstandes von einer Signaländerung des jeweils anderen Signals, oder die charakteristische Signaländerung in nur einem der Signale erfolgt.

2. Vorrichtung nach Anspruch 1, **dadurch gekennzeichnet, daß** die Ultraschalleinheit so eingerichtet ist, daß die zweite Position außerhalb des Gefäßes und des Fluidstroms liegt.

3. Vorrichtung nach Anspruch 1 oder 2, **dadurch gekennzeichnet, daß** der Mindest-Zeitabstand drei msec. beträgt.

4. Vorrichtung nach einem der Ansprüche 1 bis 3, **dadurch gekennzeichnet, daß** die Ultraschalleinheit so ausgebildet ist, daß das zweite Signal zu einem gegenüber dem ersten Signal verschiedenen Zeitpunkt entsprechend einer unterschiedlichen Eindringtiefe des Ultraschallsignals in den Körper aus einem gemeinsamen Sendesignal erzeugt wird.

5. Vorrichtung nach einem der Ansprüche 1 bis 3, **dadurch gekennzeichnet, daß** das erste Signal aus einer ersten Ultraschall-Sondeneinheit als Signalquelle und das zweite Signal aus einer zweiten Ultraschall-Sondeneinheit als weiterer Signalquelle erzeugt wird.

6. Vorrichtung nach einem der Ansprüche 1 bis 5, **dadurch gekennzeichnet, daß** die Ultraschalleinheit so ausgebildet ist, daß die zweite Position im Kopfbereich und in einer Tiefe, bezogen auf einen zugehörigen Ultraschallwandler, von zwischen etwa 30 und 35 mm liegt, und die erste Position in einer Tiefe größer als 40 mm liegt.

7. Vorrichtung nach einem der Ansprüche 1 bis 6, **dadurch gekennzeichnet, daß** die Ultraschalleinheit empfangsseitig mehrkanalig mit einer Mehrzahl von zum Ausgeben von diskreten Signalzuständen des ersten und/oder des zweiten Signals ausgebildeten Empfangseinheiten ausgebildet ist, die als Reaktion auf ein gemeinsames Sendesignal zeitlich versetzt gesteuert werden können.

8. Vorrichtung nach einem der Ansprüche 1 bis 7, **dadurch gekennzeichnet, daß** die Detektoreinheit eine Verarbeitung des ersten und des zweiten Signals zum Erfassen des Embolus im Zeitbereich vornimmt.

9. Vorrichtung nach einem der Ansprüche 1 bis 7, **dadurch gekennzeichnet, daß** die Detektoreinheit so ausgebildet ist, daß eine Verarbeitung des ersten und des zweiten Signals zum Erfassen des Embolus auf der Basis mindestens eines in den Frequenzbereich transformierten Signals erfolgt.

10. Verfahren zur Emboliedetektion mittels an einem Fluidstrom in einem Körper reflektierten Ultraschallsignalen, insbesondere zum Betreiben der Vorrichtung nach einem der Ansprüche 1 bis 9, mit den Schritten:
- periodisches Abstrahlen von Ultraschall-Gefäßüberwachungssignalen über eine geeignet eingerichtete Sonde,
- Empfangen und Aufbereiten der an einem Fluidstrom in einem zu beobachtenden Gefäß reflektierten Signale und
- Auswerten der aufbereiteten Signale und Ausgeben eines Erfassungssignals für einen erfaßten Embolus als Reaktion auf die empfangenen Signale,
- zusätzliches Empfangen eines an einer Position im Körper außerhalb des zu beobachtenden Gefäßes reflektierten Referenzsignals und
- Vergleichen des Referenzsignals mit den an den Fluidstrom reflektierten Signalen und Bestimmen eines Embolus als Reaktion auf das Vorliegen einer einem Embolus entsprechenden, charakteristischen Signaländerung in dem Referenzsignal und/oder als Reaktion auf einen zeitlichen Abstand zwischen der charakteristischen Signaländerung des Referenzsignal und einer entsprechenden Signaländerung der am Fluidstrom reflektierten Signale.

## Claims

1. Device for detecting emboli by means of ultrasonic signals reflected at a fluid flow in a body, comprising an ultrasonic unit designed to periodically generate ultrasonic monitoring signals for a suitably designed ultrasonic transmitting device and to receive a first signal reflected at a first position corresponding to the fluid flow in a vessel, a downstream signal evaluation unit for processing and visually and/or acoustically outputting the reflected first signal and a detector unit cooperating with the signal evaluation unit and designed to detect an embolus in the fluid flow and to output a detection signal as a reaction thereto, the ultrasonic unit being designed to additionally receive a second signal from the ultrasonic transmitting device reflected at a second position in the body, **characterised in that** the detector unit for detecting an embolus as a reaction to the first and second signals is designed in such a manner that the detection signal is only output if a characteristic change in one of the first and second signals corresponding to a possible embolus takes place outside a minimum time interval from a change in the other respective signal or the characteristic change takes place in only one of the signals.

2. Device according to claim 1, **characterised in that** the ultrasonic unit is designed in such a manner that the second position is outside the vessel and the fluid flow.

3. Device according to claim 1 or claim 2, **characterised in that** the minimum time interval is three msec.

4. Device according to one of claims 1 to 3, **characterised in that** the ultrasonic unit is designed in such a manner that the second signal is generated from a common transmission signal at a different time from the first signal corresponding to a different depth of penetration of the ultrasonic signal into the body.

5. Device according to one of claims 1 to 3, **characterised in that** the first signal is generated from a first ultrasonic probe unit as a signal source and the second signal is generated from a second ultrasonic probe unit as a further signal source.

6. Device according to one of claims 1 to 5, **characterised in that** the ultrasonic unit is designed in such a manner that the second position is in the head region and at a depth of between approximately 30 and 35 mm in relation to an associated ultrasonic transducer and the first position is at a depth of greater than 40 mm.

7. Device according to one of claims 1 to 6, **characterised in that** the ultrasonic unit is designed at the receiving end in a multi-channel manner with a plurality of receiver units which are designed to output discrete signal states of the first and/or second signals and can be controlled in a time-displaced manner as a reaction to a common transmission signal.

8. Device according to one of claims 1 to 7, **characterised in that** the detector unit processes the first and second signals in order to detect the embolus in the time range.

9. Device according to one of claims 1 to 7, **characterised in that** the detector unit is designed in such a manner that the first and second signals are processed in order to detect the embolus on the basis of at least one signal transformed into the frequency range.

10. Method of detecting emboli by means of ultrasonic signals reflected at a fluid flow in a body, in particular for operating the device according to one of claims 1 to 9, comprising the steps:
- periodically emitting ultrasonic vessel-monitoring signals by means of a suitably designed probe,
- receiving and processing the signals reflected at a fluid flow in a vessel to be observed,
- evaluating the processed signals and outputting a detection signal for a detected embolus as a reaction to the received signals,
- additionally receiving a reference signal reflected at a position in the body outside the vessel to be observed, and
- comparing the reference signal with the signals reflected at the fluid flow and determining an embolus as a reaction to the presence of a characteristic change in the reference signal corresponding to an embolus and/or as a reaction to a time interval between the characteristic change in the reference signal and a corresponding change in the signals reflected at the fluid flow.

## Revendications

1. Dispositif de détection d'embolies au moyen de signaux à ultrasons réfléchis sur un écoulement de fluide dans un corps, avec
une unité à ultrasons, réalisée pour générer périodiquement des signaux de surveillance à ultrasons, pour un dispositif émetteur d'ultrasons équipé de manière appropriée et pour recevoir un premier signal, réfléchi dans un vaisseau, en une première position, de manière correspondant à l'écoulement de fluide,
avec une unité d'évaluation de signaux, branchée en aval de celle-ci, pour la préparation et l'émission visuelle et/ou acoustique du premier signal réfléchi, et avec une unité de détection coopérant avec l'unité d'évaluation de signaux, unité de détection réalisée pour détecter une embolie dans un écoulement de fluide, et pour fournir un signal de détection en réaction à cela,
où l'unité à ultrasons est équipée pour recevoir en plus un deuxième signal, réfléchi en une deuxième position dans le corps, du dispositif d'émission d'ultrasons,
**caractérisé en ce que**
l'unité de détection est équipée pour détecter une embolie, en tant que réaction au premier et au deuxième signal, de manière à ce qu'une émission du signal de détection ne se fasse que lorsqu'une variation de signal caractéristique, correspondant à une éventuelle embolie, d'un des premiers et deuxièmes signaux, se fasse hors d'un intervalle de temps minimal, sous l'effet d'une modification de signal, chaque fois de l'autre signal, ou bien si la variation de signal caractéristique se fasse uniquement dans l'un des signaux.

2. Dispositif selon la revendication 1, **caractérisé en ce que** l'unité à ultrasons est équipée de manière que la deuxième position se situe à l'extérieur du vaisseau et de l'écoulement de fluide.

3. Dispositif selon la revendication 1 ou 2, **caractérisé en ce que** l'espace, par un intervalle de temps minimal, est de trois msec.

4. Dispositif selon l'une des revendications 1 à 3, **caractérisé en ce que** l'unité à ultrasons est réalisée de manière que le deuxième signal soit produit à un instant différent par rapport au premier signal, de manière correspondant à une profondeur de pénétration différente du signal à ultrasons dans le corps, à partir d'un signal d'émission commun.

5. Dispositif selon l'une des revendications 1 à 3, **caractérisé en ce que** le premier signal est produit à partir d'une première unité à sonde à ultrasons, servant de source de signal, et le deuxième signal est produit à partir d'une deuxième unité à sonde à ultrasons, servant de source de signal supplémentaire.

6. Dispositif selon l'une des revendications 1 à 5, **caractérisé en ce que** l'unité à ultrasons est réalisée de manière que la deuxième position soit située dans la zone de tête et à une profondeur, en se référant à un convertisseur à ultrasons afférent, entre environ 30 et 35 mm, et la première position soit située à une profondeur supérieure à 40 mm.

7. Dispositif selon l'une des revendications 1 à 6, **caractérisé en ce que** l'unité à ultrasons est réalisée côté réception, en comprenant plusieurs canaux, avec une pluralité d'unités de réception, réalisées pour émettre des états de signaux discrets du premier et/ou du deuxième signal, qui peuvent être commandés de façon décalée temporellement, en réaction à un signal d'émission commun.

8. Dispositif selon l'une des revendications 1 à 7, **caractérisé en ce que** l'unité de détecteur effectue un traitement du premier et du deuxième signal pour appréhender l'embolie dans le domaine temporel.

9. Dispositif selon l'une des revendications 1 à 7, **caractérisé en ce que** l'unité de détecteur est réalisée de manière à effectuer un traitement du premier et du deuxième signal, pour appréhender l'embolie sur la base d'au moins un signal ayant été transformé dans le domaine fréquentiel.

10. Procédé de détection d'embolies au moyen de signaux à ultrasons, réfléchis en un écoulement de fluide dans un corps, en particulier pour le fonctionnement du dispositif selon l'une des revendications 1 à 3, comprenant les étapes consistant à :
- émettre périodiquement par rayonnement des signaux à ultrasons de surveillance de vaisseaux, par l'intermédiaire d'une sonde équipée de manière appropriée,
- recevoir et traiter les signaux réfléchis sur un écoulement de fluide se faisant dans un vaisseau à examiner,
- évaluer les signaux traités et émettre un signal de détection pour la détection d'une embolie, en réaction aux signaux reçus,
- recevoir en plus un signal de référence, réfléchi en une position dans le corps, à l'extérieur du vaisseau à examiner, et
- comparer le signal de référence aux signaux réfléchis sur l'écoulement de fluide, et détecter une embolie en réaction à la présence d'une variation de signal caractéristique, correspondant à une embolie, dans le signal de référence et/ou en réaction à un espacement temporel entre la variation de signal caractéristique du signal de référence et une variation de signal correspondante des signaux réfléchis sur l'écoulement de fluide.
